# EUROPEAN PATENT SPECIFICATION

(11) **EP 2 247 535 B1**
(45) Date of publication and mention of the grant of the patent: **02.03.2016**
(21) Application number: 08865566.7
(22) Date of filing: 17.12.2008
(51) Int. Cl.: C02F 1/04, C02F 1/26, B01D 3/14, B01D 17/02, C07C 37/68, C07C 37/72, C07C 37/74, C07C 39/04, C02F 101/32, C02F 101/34

(54) **PROCESS FOR RECOVERY OF ORGANIC COMPOUNDS FROM A WASTEWATER STREAM**
VERFAHREN ZUR RÜCKGEWINNUNG VON ORGANISCHEN VERBINDUNGEN AUS EINEM ABWASSERSTROM
PROCEDE DE SUPPRESSION D'HYDROCARBURES DEPUIS UN FLUX AQUEUX

(30) Priority: 20.12.2007 EP 07150221
(43) Date of publication of application: 10.11.2010
(73) Proprietor: Borealis Technology Oy, 06201 Porvoo (FI)
(72) Inventor: AARNI-SIRVIÖ, Maarit, FI-00850 Helsinki (FI); SYRI, Jari, FI-06400 Porvoo (FI)
(74) Representative: Campbell, Neil Boyd
(86) International application number: PCT/FI2008/050748
(87) International publication number: WO 2009/080875

(56) References cited:
- EP-A1- 0 717 024
- EP-A1- 1 256 561
- WO-A-2005/102936
- WO-A1-2004/046039
- US-A1- 2002 066 661
- PINTO R T P ET AL: "Strategies for recovering phenol from wastewater: thermodynamic evaluation and environmental concerns" FLUID PHASE EQUILIBRIA, ELSEVIER, vol. 228-229, 1 February 2005 (2005-02-01) , pages 447-457, XP004870814 ISSN: 0378-3812

## Description

### Background of the Invention

### Field of the Invention

The present invention concerns a process for the removal of organic compounds from a wastewater stream. The process is used to remove particularly heavy organic compounds from an aqueous solution obtained from a phenol production process.

### Description of Related Art

Phenol and acetone are commonly manufactured through a cumene procedure, wherein cumene is oxidized to cumene hydroperoxide (CHP) and the resulting oxidation product mixture is concentrated and subjected to a cleavage reaction. Subsequently, the cleavage product mixture is conducted to a distillation section, wherein the main products of the cleavage reaction, i.e. phenol and acetone, are first separated and then purified through a series of distillation steps or other purification steps.

The cleavage product mixture obtained in the mentioned cleavage reaction is acidic due to its concentration of added acid catalyst. When neutralizing this acid, an aqueous solution containing an alkaline component is generally used. Using an aqueous solution will cause a portion of the main products and by-products of the process to dissolve in the water, thereby causing contamination of the water purification systems. Typically these products, together with heavy organic compounds, end up in the water in process sections such as cleavage product neutralization, recovery of organic compounds and also when washing equipment containing organic compounds.

To remove these compounds from the wastewaters before conducting these wastewaters to the regular water purification systems, other equipment has been developed, one type of which is known as the dephenolation column, which is a distillation column for distilling wastewater to remove components, such as phenol. One weakness of this dephenolation column, or stripper, is that heavy organic compounds in the wastewater going to the dephenolation column will cause fouling of e.g. trays and pre-hcaters, whereby these surfaces need to be cleaned repeatedly.

To avoid this, there is a demand for further procedures or alternative procedures for removing the organic compounds from the aqueous solution.

In the prior art, as an alternative to said distillation, which is described for example in GB 707 173, dephenolisation of wastewaters has been effected by extraction, such as in US 6,071,409, EP 0 758 636, DE 1 042 599 and GB 1 426 606. In many cases, the phenol-containing wastewaters are salt containing aqueous solutions derived from processes for manufacturing bisphenol A, such as in WO 2005/102936, wherein an aqueous solution obtained by distillation of the reaction mixtures from a process for producing bisphenol A is extracted using an organic extraction agent, which is somewhat soluble in water, whereby the phenol dissolves in the extraction agent and the phenol deprived wastewater is distilled in order to remove the dissolved extraction agent. Similarily, in US 4,374,283, as a further procedure, in addition to distillation, a water-soluble extraction agent is used.

EP-A-1256561 discloses a process for the reduction of the salt content of high boiling fractions arising from the production of phenol from cumene comprises intensively mixing the high boiling fraction with an extraction agent comprising an aqueous phase of 0.1-10 wt.% acid as well as at least one organic liquid as a diluent followed by separation of the aqueous phase by liquid/liquid separation. The process is useful for the recovery of phenol from high boiling fractions containing up to 5 wt.% phenol.

In addition to removing phenol, procedures for removing other by-products and impurities would be beneficial, whereby in many cases a simple distillation is not efficient.

Thus, there is a need for a simple and efficient process for preventing heavy organic compounds from fouling surfaces, such as trays and preheaters, and for recovering at least a portion of the valuable products of the process, such as phenol, which process does not require complex equipment and, thus, expensive investments. Using the present invention, among others, the extent of the requirements for the control of the contents of organic compounds in the wastewaters of the process for manufacturing phenol and acetone is reduced.

### Summary of the Invention

It is an aim of the present invention to provide an efficient process for recovering organic compounds from a wastewater stream.

It is an aim of the present invention to provide a purification process for decreasing the concentration of particularly heavy organic compounds in a wastewater stream from the production of phenol.

These and other objects, together with the advantages thereof over known processes and apparatuses, are achieved by the present invention, as hereinafter described and claimed.

The present invention concerns a process for the recovery of organic compounds from a wastewater stream, which process comprises conducting the wastewater stream containing heavy organic compounds from the purification section of a phenol production system following the cleavage and neutralization sections into a separation system and separating the water of the wastewater stream from at least a portion of the other components.

More specifically, the process of the present invention is defined by the appended claims.

Considerable advantages are obtained by means of the invention. Thus, the present invention provides a process for recovering organic compounds from a wastewater stream, whereby heavy organic compounds are separated in a first step, thereby preventing fouling in the second step. When the second step is carried out in a distillation column, the decreased load of heavy organic compounds prevents fouling in the column. Furthermore, when used in a process for producing phenol and acetone, the present invention provides products with a higher purity compared to a production procedure with a simpler dephenolation process or no dephenolation process at all. In addition to being efficient in removing heavy organic compounds from the wastewaters, the present invention decreases the amounts of light compounds, which may be, for example, acetaldehyde, propionaldehyde, 2,2-dimethyloxirane, mesityl oxide, methyl ethyl ketone, benzene diacetyl and acetoin.

Next, the invention will be described more closely with reference to the attached drawing and a detailed description.

### Brief Description of the Drawing

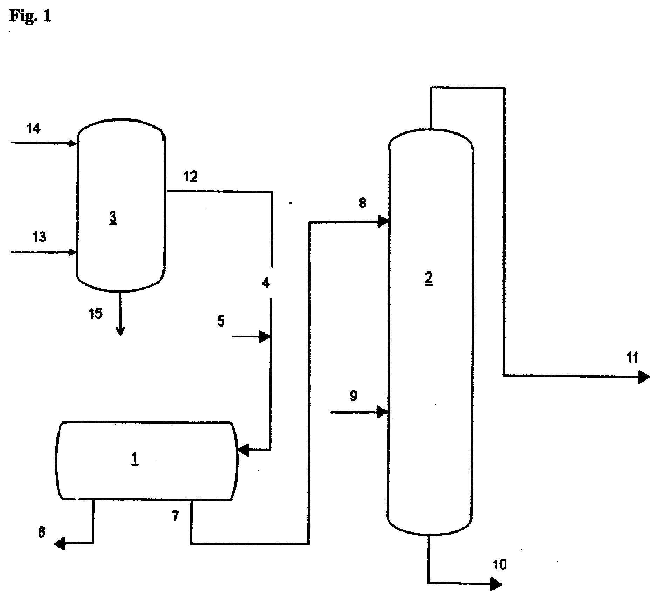

Figure 1 is a schematic drawing of a preferred embodiment of the apparatus of the present invention.

### Detailed Description of the Preferred Embodiments of the Invention

The present invention concerns a process for the recovery of organic compounds from a wastewater stream resulting from the introduction of an aqueous solution to a neutralized cleavage product mixture, which process comprises adding an eluent to the wastewater stream, thereby forming an aqueous mixture, subsequently conducting the aqueous mixture into a separation vessel (1), wherein it is allowed to settle into two phases, which form a stream of organic compounds and a water stream, collecting the stream of organic compounds, conducting the water stream to the separation column (2) for further separation, separating the water stream in the column using heat, e.g. in the form of steam, into an overhead portion and a bottoms portion, and collecting the overhead portion as well as the bottoms portion.

In the context of the present invention, the term "wastewater stream" is intended to mean an aqueous solution containing water, organic compounds and one or more further organic or inorganic component, whereas the term "aqueous mixture" is intended to mean a wastewater stream further containing an amount of added eluent. Further, the term "acidified mixture" is intended to mean any mixture, preferably a wastewater stream or an aqueous mixture, whose pH has been adjusted to a desired level using an acid.

When separating the aqueous mixture into two phases, it divides into a "stream of organic compounds", i.e., the organic phase of the separated aqueous mixture, which in addition to said added eluent contains compounds, such as cumene, acetone, phenol, heavy organic compounds, methanol, acetaldehyde, propionaldehyde, benzene, ethanol, alpha-methyl styrene and mesityl oxide, and a "water stream", i.e., the aqueous phase of the separated aqueous mixture, containing, for example, water, salts and, possibly, a portion of said organic compounds. Before this separation, an optional process step may be performed, wherein a fraction of "heavy organic compounds" is removed from the wastewater stream or the acidified mixture, which heavy organic compounds are organic compounds with a larger density compared to the density of water (e.g. AMS dimers), whereby they settle as a bottom phase during phase separation.

Further, in the context of the present invention, the term "distillation" is intended to mean a method for separating chemical components based on the differences in their volatilities. The mixture conducted to the distillation column, wherein the distillation takes place, is separated into a distillate, i.e., an overhead portion, containing "light" or "low-boiling" components, i.e., components that vaporize under the conditions prevailing in the column, and an unevaporated fraction, i.e., a bottoms portion, containing "heavy" or "high-boiling" components, i.e., components that remain in liquid form under the conditions prevailing in the column. The identities of the compounds included in the heavy components or in the light components depend on the prevailing conditions in the distillation column, such as the temperature and the pressure. The term "evaporation" is intended to mean any process where at least a portion of a mixture of components is converted from a liquid phase to a vapor phase, generally by heating.

The apparatus of the present invention for removing organic compounds from a wastewater stream contains the following parts (Fig. 1)

| | |
|---|---|
| 1 | separation vessel |
| 2 | separation column |
| 3 | acidification vessel |

Preferably, the separation vessel 1 further comprises the following parts:

| | |
|---|---|
| 4 | aqueous mixture inlet |
| 5 | eluent inlet |
| 6 | outlet for organic compounds |
| 7 | aqueous outlet |

Preferably, the separation column 2 further comprises the following parts:

| | |
|---|---|
| 8 | aqueous inlet |
| 9 | steam inlet |
| 10 | bottoms outlet |
| 11 | overhead outlet |

Preferably, the acidification vessel 3 further comprises the following parts:

| | |
|---|---|
| 12 | outlet for acidified mixture |
| 13 | inlet for wastewater stream |
| 14 | acid inlet |
| 15 | outlet for heavy organic compounds |

Thus the apparatus for separating the components of a wastewater stream comprises a separation vessel 1, wherein an aqueous mixture may be allowed to settle into two phases, which are a stream of organic compounds and a water-stream, whereby the water stream contains, among others, phenol and a phenol-water azeotrope, a separation column 2 positioned downstream from the separation vessel 1, in fluid communication with the vessel, for separating said water stream into a bottoms portion and an overhead portion, and an acidification vessel 3 positioned upstream from the separation vessel 1, in fluid communication with the vessel 1, for adjusting the pH of said wastewater stream to a desired level.

The separation vessel 1 preferably comprises an inlet 4 for the aqueous mixture and an inlet 5 for the eluent as well as an outlet 6 for organic compounds and an outlet 7 for the water stream. The separation column 2 preferably comprises an inlet 8 for the water stream, which inlet 8 is connected to the aqueous outlet 7 on the separation vessel 1, a steam inlet 9, an outlet 10 for the bottoms portion and an outlet 11 for the overhead portion. Likewise, the acidification vessel 3 preferably comprises an outlet 12 for the acidified mixture, which outlet 12 is connected to the aqueous mixture inlet 4 of the separation vessel 1, a wastewater inlet 13 and an acid inlet 14.

According to a preferred embodiment of the present invention, a further outlet for heavy organic compounds 15 is positioned in connection to the acidification vessel 3, preferably at the bottom of the vessel 3, through which outlet 15 a fraction containing particularly heavy organic compounds can be removed.

The process and the apparatus of the present invention may be used to separate or recover organic compounds from any wastewater stream. Preferably, the apparatus of the invention is arranged downstream from the cumene hydroperoxide cleavage section of a process for producing phenol and acetone, more preferably it is in fluid connection either with the outlet of a purification system, directly downstream from the cleavage product neutralization section of said process for producing phenol and acetone, or it is in fluid connection with the bottom of an acetone purification column, or it is in connection with both the purification system and the acetone purification column. The production process typically comprises process steps, wherein cumene is oxidized to cumene hydroperoxide (CHP) and the subsequent steps of concentrating the CHP, cleaving the CHP into phenol, acetone and other cleavage products, neutralizing, washing and desalting the cleavage products and finally separating the acetone from the phenol and purifying both desired products.

The wastewater stream to be processed according to the present invention preferably results from this mentioned process, wherein the cumene is subjected to oxidation in a series of reactors using air or concentrated oxygen as the mentioned air feed, the concentrated oxygen being a gaseous mixture containing up to 100% oxygen, preferably about 22-80% oxygen, the rest being mostly inert gases. The concentration of the CHP formed during the oxidation is increased in a series of concentration steps, after which the CHP is conducted to a cleavage reactor, wherein the concentrated oxidation product is subjected to an acid catalyzed cleavage process, the acid catalyst being any organic or inorganic acidic compound in a solution having a pH below 7, preferably having a pH of 1-5. The resulting cleavage product mixture is subsequently neutralized and subjected to desalting. Finally, the aqueous phase from the desalting process is conducted to a stripper column, wherein the organic components contained therein are recovered, and the organic phase is conducted further to the distillation section of the process for producing phenol and acetone. In the distillation section the desalted cleavage product mixture is distilled, first in order to separate a crude distillate containing, among others, acetone, water, cumene, AMS, hydroxyacetone and mesityl oxide, from a crude base product containing, among others, phenol, acetophenone, carbinol, mesityl oxide and heavy organic compounds, and further to separate impurities from the product phenol and the product acetone.

According to the present invention, organic compounds are separated from a wastewater stream in a separation vessel 1 with the help of the addition of an eluent. The purpose of the addition of eluent is to create a sufficient density difference between the organic phase and the aqueous phase for their separation. The eluent may be any essentially water-insoluble organic compound, preferably cumene, since cumene may already be a part of the process streams and does not dissolve into water in any significant amounts, whereas for example phenol forms an azeotrope with water. The amount of added eluent, such as cumene, is sufficient to give an aqueous mixture with a cumene concentration of 0.2-20 w-%, preferably 0.2-10 w-%, most preferably 0.2-5 w-%, calculated from the total amount of incoming wastewater, before separation. The eluent may be freshly added to the process streams or it may be recycled.

According to a preferred embodiment of the present invention, the extraction with eluent is performed as a simple mixing followed by decanting.

The conditions prevailing in the separation vessel 1 are close to atmospheric conditions, the temperature being 30-70°C, preferably about 40-50°C, and the pressure being 110-200kPa.

According to the a preferred embodiment of the present invention, the pH of the wastewater stream being conducted to the separation vessel 1 is maintained at a level of 4.5-6 using an acid, preferably an inorganic acid, most preferably sulfuric acid. Thus, any sodium phenate in the water is converted to phenol and sodium sulfate. Other similar alkali salts also react. Further, the pH adjustment changes the solubilities of the components of the wastewater stream, whereby for example any cumene present in the stream becomes even less soluble in water. A lower pH than the mentioned level may cause equipment corrosion. The concentration of salt in the wastewater stream depends on the treatments that are carried out on the stream before conducting it to the vessel 1. However, the salt concentration (Na⁺ and K⁺ ions) is not allowed to increase above 1000 ppm. Thus, no salts or alkalis are required to be added to the wastewater or to the eluent. The amount of organic compounds in the wastewater stream, before the addition of eluent, is generally about 2-7 w-%, preferably 3-5 w-%, most preferably 3.5-4 w-%. This pH adjustment is preferably carried out in the acidifying vessel 3 of the apparatus of the invention.

According to another preferred embodiment, an optional step of phase separation is carried out directly on the acidified mixture, before the addition of an eluent. Preferably, the wastewater stream having an adjusted pH is allowed to settle, whereby a heavy fraction containing heavy organic compounds separates from the stream, which heavy fraction is subsequently removed. If this step is not carried out, the heavy organic compounds will be conducted to the separation vessel 1 with the wastewater stream (the acidified mixture) and the eluent, whereby said heavy organic compounds will be essentially separated from the water together with other organic compounds in the subsequent steps.

After the eluent has been added to the wastewater stream, thereby forming an aqueous mixture, the formed mixture is allowed to settle, whereby an aqueous phase and an organic phase are formed.

According to a preferred embodiment of the present invention, the mentioned separation process is used as a part of the process for producing phenol and acetone, whereby the separated organic phase contains the eluent and further organic compounds, while the aqueous phase contains the phenol and a phenol-water azeotrope.

The organic phase, i.e., the organic stream containing eluent and one or more organic compounds, is collected from the separation vessel 1, while the aqueous phase, i.e., the water stream, is conducted further to the separation column 2. The function of this column 2, when being a part of a phenol production procedure, is based on the fact that phenol and water form an azeotrope, which has a boiling point below the ones of water and phenol. The azeotrope can therefore be separated from water using stripping. The azeotrope also has the characteristic that its relative volatility in comparison with water increases when the pressure increases. During this stripping process, at least a portion of the phenol present in the water stream is separated from the stream, and may, for example, be conducted back to the process, preferably to the stream being conducted to the distillation section of the process for producing phenol and acetone.

The stripping in the column 2 may be performed either as a steam stripping or as a conventional distillation, preferably as a steam stripping.

According to an embodiment of the present invention, the conditions in the column 2 are maintained at levels that give an overhead portion that consists to a concentration of 75-95 w-%, preferably about 90 w-%, of the mentioned azeotrope, whereas the concentration of phenol in the overhead portion under these conditions is about 3-10 w-%, preferably about 5-8 w-%, and in the bottoms portion about 50-150ppm, preferably about 90-100ppm. In addition to the small amount of phenol in the bottoms portion, it contains mostly water.

According to a preferred embodiment of the present invention, the separation column 2 is a distillation column 2 operated with reflux using steam, which preferably is recycled steam, of a temperature around 100-200°C, preferably about 150°C. The pressure in the column 2 is maintained close to atmospheric pressure, preferably slightly above atmospheric pressure, more preferably at about 110-300kPa.

## Claims

1. A process for the recovery of organic compounds from a wastewater stream resulting from the introduction of an aqueous solution to a neutralized phenolic cleavage product mixture and from the purification of acetone, which process comprises
- adjusting the pH of the wastewater stream which comprises 2 to 7 wt% organic compounds to less than 6, preferably to 4-6, before the addition of the eluent;
- adding cumeme as an eluent to the wastewater stream, thereby forming an aqueous mixture such that the eluent forms 0.2 to 5 wt% of the wastewater stream;
- subsequently conducting the aqueous mixture into a separation vessel (1), wherein it is allowed to settle into two phases, which form a stream of organic compounds containing eluent and further organic compounds and a water stream, said water stream containing phenol and a phenol/water azeotrope,
- subsequently collecting the stream of organic compounds; and
- conducting the water stream to a separation column (2) for further separation;
- separating the water stream in the column (2) using heat into an overhead portion comprising a phenol water azeotrope portion wherein the conditions in the column (2) are maintained at levels that give an overhead portion having a concentration of 75-95 wt% of phenol and water azeotrope and 3 to 10 wt% phenol; and a bottoms portion containing 50 to 150 ppm phenol, and
- collecting the overhead portion as well as the bottoms portion.

2. The process of claim 1, **characterized by** adjusting the pH of the wastewater stream to less than 6, preferably to 4-6, using sulphuric acid, before the addition of the eluent.

3. The process of claim 1 or claim 2, **characterized by** allowing the wastewater stream having an adjusted pH to settle, and subsequently separating a heavy fraction containing heavy organic compounds.

4. The process of any preceding claim, **characterized by** adding an eluent to the wastewater stream to form an aqueous mixture containing water, the eluent and one or more organic compounds selected from heavy organic compounds, phenol, methanol, acetone, cumene, acetaldehyde, propionaldehyde, benzene, ethanol, alpha-methyl styrene and mesityl oxide.

5. The process of claim 1, **characterized by** separating the water stream in the column (2) using steam.

6. The process of claim 1 wherein the salt concentration of said wastewater stream is not allowed to increase above 1000 ppm (Na and K ions).

7. The process of claim 1, **characterized in that** the temperature in the separation vessel (1) is 30-50°C, preferably at about 40°C, and the pressure is close to atmospheric pressure, preferably about 110-200 kPa.

8. The process of any preceding claim, **characterized in that** the separation column (2) is operated with reflux using steam, such as recycled steam, at a temperature of 100-200°C, preferably about 150°C.

## Patentansprüche

1. Verfahren zum Gewinnen von organischen Verbindungen aus einem Abwasserstrom, der aus der Einführung einer wässrigen Lösung zu einer neutralisierten phenolischen Spaltproduktmischung und aus der Reinigung von Aceton herrührt, wobei das Verfahren folgendes umfasst:
- Einstellen des pH von dem Abwasserstrom, der 2 bis 7 Gew.-% organische Verbindungen umfasst, vor der Zugabe des Eluents auf weniger als 6, vorzugsweise auf 4 - 6;
- Zugeben von Cumol als ein Eluent zu dem Abwasserstrom, wodurch eine wässrige Mischung gebildet wird, sodass das Eluent 0,2 bis 5 Gew.-% des Abwasserstroms ausmacht;
- anschließendes Leiten der wässerigen Mischung in ein Trenngefäß (1), wobei ihr erlaubt wird sich in zwei Phasen zu setzen, die einen Strom aus organischen Verbindungen, die das Eluent und weitere organische Verbindungen enthalten, und einen Wasserstrom bilden, wobei der Wasserstrom Phenol und ein Phenol/Wasser-Azeotrop enthält;
- anschließendes Sammeln von dem Strom aus organischen Verbindungen; und
- Leiten des Wasserstroms zur weiteren Trennung in eine Trennkolonne (2);
- Trennen von dem Wasserstrom in der Kolonne (2), wobei Hitze verwendet wird, und zwar in einen Overhead-Teil, der einen Phenol-Wasser-Azeotropteil umfasst, wobei die Bedingungen in der Kolonne (2) auf Niveaus aufrechterhalten werden, die zu einem Overhead-Teil führen, der eine Konzentration von dem Phenol-und-Wasser-Azeotrop von 75 - 95 Gew.-% und von dem Phenol von 3 bis 10 Gew.-% hat; und in einen Bodenteil, der 50 bis 150 ppm Phenol enthält; und
- Sammeln des Overhead-Teils sowie des Bodenteils.

2. Verfahren nach Anspruch 1, **gekennzeichnet durch** das Einstellen des pH von dem Abwasserstrom vor der Zugabe des Eluents auf weniger als 6, vorzugsweise auf 4 - 6, wobei Schwefelsäure verwendet wird.

3. Verfahren nach Anspruch 1 oder 2, **dadurch gekennzeichnet, dass** dem Abwasserstrom, der einen eingestellten pH hat, erlaubt wird sich zu setzen, und **gekennzeichnet durch** das anschließende Abtrennen von einer schweren Fraktion, die schwere organische Verbindungen enthält.

4. Verfahren nach einem der vorhergehenden Ansprüche, **gekennzeichnet durch** das Zugeben von einem Eluent zu dem Abwasserstrom, um eine wässrige Mischung zu bilden, die Wasser, das Eluent und ein oder mehrere organische Verbindungen enthält, die aus schweren organischen Verbindungen, Phenol, Methanol, Aceton, Cumol, Acetaldehyd, Propionaldehyd, Benzol, Ethanol, alpha-Methylstyrol und Mesityloxid ausgewählt sind.

5. Verfahren nach Anspruch 1, **gekennzeichnet durch** das Trennen des Wasserstroms in der Kolonne (2), wobei Dampf verwendet wird.

6. Verfahren nach Anspruch 1, wobei die Salzkonzentration von dem Abwasserstrom nicht über 1000 ppm (Na- und K-lonen) ansteigen darf.

7. Verfahren nach Anspruch 1, **dadurch gekennzeichnet, dass** die Temperatur in dem Trenngefäß (1) 30 - 50°C ist, vorzugsweise etwa 40°C, und der Druck nahe dem atmosphärischen Druck ist, vorzugsweise etwa 110-200 kPa.

8. Verfahren nach Anspruch 1, **dadurch gekennzeichnet, dass** die Trennkolonne (2) mit Reflux unter Verwendung von Dampf, wie zum Beispiel recyceltem Dampf, bei einer Temperatur von 100 - 200°C, vorzugsweise etwa 150°C, betrieben wird.

## Revendications

1. Processus pour la récupération de composés organiques à partir d'un flux d'eaux usées résultant de l'introduction d'une solution aqueuse d'un mélange de produit de clivage phénolique neutralisé et de la purification d'acétone, lequel processus comprend :
- l'ajustement du pH du flux d'eaux usées qui comprend 2 à 7 % en poids de composés organiques à moins de 6, de préférence à 4 à 6, avant l'ajout de l'éluant ;
- l'ajout de cumène en tant qu'éluant au flux d'eaux usées, formant ainsi un mélange aqueux de sorte que l'éluant forme 0,2 à 5 % en poids du flux d'eaux usées ;
- par la suite l'amenée du mélange aqueux dans une cuve de séparation (1), dans laquelle on l'autorise à décanter en deux phases, qui forment un flux de composés organiques contenant l'éluant et des composés organiques supplémentaires et un flux d'eau, ledit flux d'eau contenant du phénol et un azéotrope de phénol / eau,
- par la suite la collecte du flux de composés organiques ; et
- l'amenée du flux d'eau à une colonne de séparation (2) pour une séparation supplémentaire ;
- la séparation du flux d'eau dans la colonne (2) en utilisant de la chaleur en une partie formant distillat de tête comprenant une partie formant azéotrope de phénol / eau dans laquelle les conditions dans la colonne (2) sont maintenues à des niveaux qui donnent une partie formant distillat de tête ayant une concentration de 75 à 95 % en poids d'azéotrope de phénol et d'eau et 3 à 10 % en poids de phénol ; et en une partie formant queue de distillation contenant 50 à 150 ppm de phénol, et
- la collecte de la partie formant distillat de tête aussi bien que de la partie formant queue de distillation.

2. Processus selon la revendication 1, **caractérisé par** l'ajustement du pH du flux d'eaux usées à moins de 6, de préférence à 4 à 6, en utilisant de l'acide sulfurique, avant l'ajout de l'éluant.

3. Processus selon la revendication 1 ou la revendication 2, **caractérisé par** l'autorisation au flux d'eaux usées ayant un pH ajusté à décanter, et par la suite la séparation d'une fraction lourde contenant des composés organiques lourds.

4. Processus selon n'importe quelle revendication précédente, **caractérisé par** l'ajout d'un éluant au flux d'eaux usées pour former un mélange aqueux contenant de l'eau, l'éluant et un ou plusieurs composés organiques sélectionnés à partir de composés organiques lourds, phénol, méthanol, acétone, cumène, acétaldéhyde, propionaldéhyde, benzène, éthanol, alphaméthyle styrène et oxyde de mésityle.

5. Processus selon la revendication 1, **caractérisé par** la séparation du flux d'eau dans la colonne (2) en utilisant de la vapeur.

6. Processus selon la revendication 1, dans lequel la concentration de sel dudit flux d'eaux usées n'est pas autorisée à augmenter au-dessus de 1 000 ppm (ions Na et K).

7. Processus selon la revendication 1, **caractérisé en ce que** la température dans la cuve de séparation (1) est de 30 à 50 °C, de préférence à environ 40 °C et la pression est proche de la pression atmosphérique, de préférence environ 110 à 200 kPa.

8. Processus selon n'importe quelle revendication précédente, **caractérisé en ce que** la colonne de séparation (2) est mise en oeuvre par reflux en utilisant de la vapeur, comme de la vapeur recyclée, à une température de 100 à 200 °C, de préférence environ 150°C.
